# EUROPÄISCHE PATENTSCHRIFT

(11) **EP 0 848 611 B1**
(45) Veröffentlichungstag und Bekanntmachung des Hinweises auf die Patenterteilung: **18.07.2001**
(21) Anmeldenummer: 96932478.9
(22) Anmeldetag: 06.09.1996
(51) Int. Cl.: A61K 31/00, A61K 31/47, A61K 9/70

(54) **TACRIN-PFLASTER**
TACRINE PATCH
TIMBRE A LA TACRINE

(30) Priorität: 07.09.1995 DE 19533089
(43) Veröffentlichungstag der Anmeldung: 24.06.1998
(73) Patentinhaber: HEXAL AG, D-83607 Holzkirchen (DE)
(72) Erfinder: SENDL-LANG, Anna, D-83607 Holzkirchen (DE); FISCHER, Wilfried, D-83607 Holzkirchen (DE)
(74) Vertreter: Boeters, Hans Dietrich, Dr.
(86) Internationale Anmeldenummer: EP9603917
(87) Internationale Veröffentlichungsnummer: WO9709050

(56) Entgegenhaltungen:
- EP-A- 0 332 147
- EP-A- 0 356 382
- EP-A- 0 499 662
- WO-A-88/02256
- WO-A-93/02669
- WO-A-94/23707

## Beschreibung

Tetrahydroaminoacridin (Tacrin) wird bei der Alzheimer-Krankheit als ein zentral wirksamer Cholinesterasehemmer verwendet. Seine Cholinesterasehemmung hält etwa 15 h an, also deutlich länger als die Halbwertszeit von Tacrin, die im Bereich von 1,5 bis 3,5 h liegt. Die tägliche Dosisempfehlung variiert zwischen 30 und 160 mg, wobei der therapeutische Blutspiegel im Bereich von 5 bis 70 ng/ml und die Bioverfügbarkeit im Bereich von 10 bis 30 % liegt. Bei Patienten, die an der Alzheimer-Krankheit leiden und über eine längere Zeit mit mehreren Tagesdosen an Tacrin behandelt werden, läßt sich eine klinische Verbesserung des Gedächtnisses und der funktionalen Autonomie beobachten.

Tacrin wird oral in Form des Hydrochlorids eingesetzt (Cognex^{R}). Da die orale Gabe von Tacrin wegen seiner kurzen Eliminationshalbwertszeit in multiplen Dosen erfolgen muß, andererseits die Freisetzung des Wirkstoffs über eine lange Zeit konstant bleiben sollte, würde eine transdermale Gabe eine effektivere Arzneimitteltherapie darstellen. Ein zusätzliches Problem stellen die stark ausgeprägte First-Pass-Metabolisierung und die stark schwankende Pharmakokinetik von Tacrin dar, die eine schlechte Einstellbarkeit der Patienten, das Auftreten von Blutplasmaspitzen sowie eine Erhöhung der Nebenwirkungen und der Toxizität vor allem auf die Leber zur Folge haben. Der hepatotoxische Effekt des Tacrins tritt bei etwa der Hälfte der Patienten auf, wobei ein Anstieg der ALT-Werte (Alaninaminotransferase) bis zum 20-fachen der oberen normalen Grenze beobachtet wird. Durch eine kontinuierliche transdermale Gabe von Tacrin könnte es zu konstanteren und niedrigeren therapeutisch wirksamen Plasmaspiegeln und somit zu geringeren Nebenwirkungen vor allem auf die Leber führen, zumal der hohe First-Pass-Effekt und die geringe Bioverfügbarkeit wegfallen sollten.

EP-B-0 332 147 beschreibt ein transdermales System mit einem Gehalt an
(a) Diphenhydramin, Tetrahydroaminoacridin, Atenolol, Tazifyllin, 2-Methoxy-4-[2-(5-methyl-1H-pyrazol-3-yl)-ethenyl]-phenol oder eines pharmazeutisch annehmbaren Salzes dieser Verbindungen als Wirkstoff,
(b) einem Propylenglycoldiester von Caprylsäure und Caprinsäure (Migliol) und
(c) Kieselsäure als Geliermittel.
Migliol erhöht in diesem transdermalen System den Substanzflux durch Mäusehaut im Vergleich zu einer Formulierung ohne Migliol. Fakultativ kann ein Permeationsbeschleuniger in Form eines Lösungsmittels zugegeben werden, beispielsweise Alkohol. Alkohol reizt jedoch die Haut, trocknet sie aus und schädigt sie. Der Einsatz von Ethanol ist kritisch zu beurteilen, da er die natürliche Lipidschicht der Haut stört und sie austrocknet und reizt. Hinzu kommt, daß nach EP-B-0 499 662 die Freisetzungskontrolle des Wirkstoffs beim Pflaster gemäß EP-B-0 332 147 nicht optimal ist.

So wird nach der EP-B-0 499 662 ein zweilagiges Verbundlaminat als transdermales System vorgesehen, das aus
A) einer Schicht I mit einer Komponente A, die aus einem Lösungsmittel und einem aktiven Wirkstoff in einem teilweise ausgehärteten Elastomeren besteht, und
B) einer Schicht II auf der Schicht I besteht, die eine Komponente B umfaßt, die aus einem Lösungsmittel und einem Wirkstoff in einem makroporösen Formkörper mit einer Porengröße von 10 bis 100 *µ*m besteht.
Als Beispiel für einen Wirkstoff wird unter anderem 1,2,3,4-Tetrahydro-9-acridinamin (THA = Tacrin) genannt. Als Lösungsmittel für die Komponenten A und B werden Alkohole vorgeschlagen, beispielsweise Ethanol. Durch Ethanol wird jedoch wiederum die Haut ausgetrocknet, gereizt und geschädigt.

Zudem ist das aus EP-B-0 499 662 bekannte schwammartige transdermale System dick und unflexibel und somit bei Anwendung durch den Patienten nicht sehr praktikabel, da das System sich durch seine Höhe exponiert, leicht ungewollt entfernbar ist und Körperbewegungen nicht sehr gut mitmacht.

Das aus EP-B-0 499 662 bekannte transdermale System wird dadurch hergestellt, daß man
(a) einen Wirkstoff mit einem Lösungsmittel und einer härtbaren Elastomerkomponente mischt,
(b) die erhaltene Komponente A zu einer teilgehärteten Schicht I gießt,
(c) einen Wirkstoff in einem Lösungsmittel löst,
(d) die erhaltene Lösung in einen makroporösen Formkörper einführt und Komponente B erhält, die die Schicht II bildet,
(e) Schicht II auf Schicht I laminiert und gegebenenfalls das anfallende Laminat zu Pflastern gewünschter Größe zuschneidet.
Diese Herstellung ist schwierig nachvollziehbar, kompliziert und führt außerdem zu wenig reproduzierbarem Blutspiegel, möglicherweise durch die schlecht kontrollierbare Teilhärtung des Elastomeren. Wenn außerdem bei diesem sehr kostenaufwendigen Verfahren über mehrere Minuten eine erhöhte Temperatur von beispielsweise 160 °C angewendet wird, kann dadurch der Wirkstoff abgebaut werden. Neben der Frage, wie eine Aluminiumfolie auf den makroporösen Schwamm aufzulaminieren ist, bleibt die Frage offen, wie das transdermale System am Patienten appliziert werden kann, da offensichtlich kein klebendes Element vorgesehen ist.

Eine Aufgabe der Erfindung ist es daher, ein reproduzierbar und kostengünstig herzustellendes, hautverträgliches und anwenderfreundliches transdermales System vorzusehen. Dieses transdermale System soll eine effektive Arzneimitteltherapie gewährleisten, bei der die Freisetzung des Wirkstoffs über eine lange Zeit konstant bleibt. Es soll ferner konstante, niedrige und therapeutisch wirksame Plasmaspiegel garantieren und somit zu geringeren Nebenwirkungen führen, vor allem auf die Leber.

Dazu wird erfindungsgemäß ein transdermales System beziehungsweise Pflaster mit einer äußeren Abdeckschicht (outer covering), einer selbstklebenden Matrix (selfadhesive matrix) und einer entfernbaren Schutzschicht (protective liner) vorgesehen, bei dem die Matrix
- Tacrin oder eines seiner pharmazeutisch verträglichen Salze als Wirkstoff neben
- einem hydrophilen schwerflüchtigen Lösungsmittel und/oder
- einem lipophilen schwerflüchtigen Lösungsmittel enthält.

In Hinblick auf die Anstrengungen, die nach EP-B-0 499 662 unternommen wurden, um ein Pflaster mit Tacringehalt vorzusehen, konnte nicht erwartet werden, daß sich mit einem erfindungsgemäßen Pflaster befriedigende Ergebnisse erreichen lassen würden. Das erfindungsgemäße Pflaster läßt sich darüberhinaus kostengünstig, reproduzierbar und ohne größere Wärmebelastung herstellen. Das erfindungsgemäße Pflaster ist dünn, hautverträglich, flexibel und sehr gut auf die Haut applizierbar.

Das erfindungsgemäße Pflaster unterscheidet sich von einem Pflaster nach EP-B-0 332 147 dadurch, daß der Wirkstoff nicht aus einem Gel unter Verwendung von Kieselsäure als Gelierungsmittel austritt, vielmehr aus einer selbstklebenden Matrix ohne Gehalt an Kieselsäure. Mit dem erfindungsgemäßen Pflaster wird auch bewußt darauf verzichtet, den Wirkstoff wie bei EP-B-0 499 662 in zwei verschiedenen Schichten vorzusehen, die ein Laminat bilden. Vielmehr wird der Wirkstoff in einer einzigen Schicht des erfindungsgemäßen Pflasters vorgesehen, bei der es sich um eine selbstklebende Matrix handelt.

Nachdem für orale Applikation die empfohlene tägliche Dosis im Bereich von 30 bis 160 mg und der therapeutische Blutspiegel im Bereich von 5 bis 70 ng/ml liegen, war nicht zu erwarten, daß ein Pflaster zur transdermalen Applikation mit einer vergleichbaren Wirkstoffmenge beladbar und vergleichbare Blutspiegel erzielbar sein würden.

Das erfindungsgemäße Pflaster kann durch einen Gehalt von bis zu 70 Gew.-% (bezogen auf das Matrix-Gewicht) an einem Lösungsmittel aus der durch hydrophile schwerflüchtige Lösungsmittel, lipophile schwerflüchtige Lösungsmittel und deren Gemische gebildeten Gruppe gekennzeichnet sein.

Eine weitere Aufgabe der Erfindung ist es, ein reproduzierbar und kostengünstig herzustellendes und anwenderfreundliches transdermales System vorzusehen, das die Vorteile eines Reservoir-Pflasters bieten soll und bei dem sich die bei Gegenwart von niedermolelularen Alkoholen, wie Ethanol, auftretenden Probleme nicht wie üblich zeigen oder durch Vorteile kompensiert werden.

Dazu wird erfindungsgemäß ein transdermales System beziehungsweise Pflaster mit einer äußeren Abdeckschicht (outer covering), einem Reservoir, einem Klebeelement für den Hautkontakt des Pflasters und einer entfernbaren Schutzschicht (protective liner) vorgesehen, wobei das Reservoir
- Tacrin oder eines seiner pharmazeutisch verträglichen Salze als Wirkstoff neben
- einem Lösungsmittel aus der durch hydrophile schwerflüchtige Lösungsmittel, hydrophobe schwerflüchtige Lösungsmittel, einwertige C₂₋₄-Alkohole, Ethylenglykol und deren Gemische gebildeten Gruppe enthält.

Reservoir-Pflaster gehören zum Stand der Technik. Lediglich beispielsweise sei auf WO-A1-89/09 051, WO-A1-94/23 707, EP-B1-0 404 807 und EP-A1-0 406 488 verwiesen.

Für die genannte Ausführungsform kann eine Membran vorgesehen werden, insbesondere eine die Wirkstoffpermeation steuernde Membran.

Bei der genannten Ausführungsform kann das Reservoir durch die Abdeckschicht und die Membran oder durch eine Matrix gebildet werden.

Für das erfindungsgemäße Pflaster kann man eine Matrix auf Polyacrylatbasis, Silikonbasis oder Polyisobutylenbasis vorsehen. Derartige Matrices sind im Stand der Technik vorgegeben. Lediglich beispielhaft sei für eine Polyacrylat-Matrix auf DE-B 3 933 460, eine Silikon-Matrix auf DE-A-4 339 400 und eine Polyisobutylen-Matrix auf EP-A-0 186 019 verwiesen.

Das Klebeelement kann in Form einer das Reservoir (sofern eine Membran fehlt) oder einer die Membran vollständig oder lediglich an ihrer Peripherie ringförmig abdeckenden Schicht vorgesehen sein. Vorzugsweise besteht das Klebeelement aus einem druckempfindlichen Klebemittel auf Silikon-Basis.

Das erfindungsgemäße Pflaster kann zusätzlich α-Tocopherol oder ein α-Tocopherol-Derivat enthalten. Dadurch kann der kritischen Hautverträglichkeit bei der Verwendung niedermolekularer Alkohole begegnet werden.

Auch kann das erfindungsgemäße Pflaster ein viskositätserhöhendes Mittel enthalten, beispielsweise hochdisperses Siliciumdioxid oder Hydroxypropylcellulose.

Für ein erfindungsgemäßes Pflaster kann man als hydrophiles schwerflüchtiges Lösungsmittel beispielsweise Propylenglykol, 1,2-Pentandiol, Glycerin, hydrophiles Cetiol oder Transcutol verwenden. Propylenglykol und Glyzerin werden bereits mit EP-B-0 499 662 vorgeschlagen, allerdings für das vorstehend diskutierte abweichende transdermale System.

Ferner kann man für das erfindungsgemäße Pflaster als lipohiles schwerflüchtiges Lösungsmittel beispielsweise Copherol, Propylenglykol-Dicaprylat/Dicaprat, wie Migliol, Isopropylmyristat oder lipophiles Cetiol, beispielsweise Cetiol HE, verwenden. Isopropylmyristat wird bereits mit EP-B-0 332 147 und Migliol wird bereits mit EP-B-0 332 147 und EP-B-0 499 662 vorgeschlagen, jeweils jedoch für die vorstehend diskutierten abweichenden transdermalen Systeme.

Beim erfindungsgemäßen Pflaster überrascht besonders, daß eine Kombination von hydrophilem schwerflüchtigen Lösungsmittel mit lipophilem schwerflüchtigen Lösungsmittel zu einer deutlich höheren Hautpermeationsrate als mit jedem Lösungsmittel für sich allein führt.

Nachstehend wird die Erfindung durch Beispiele näher erläutert.

### Beispiel 1

Tacrinbase wird in Ethanol gelöst, die Lösung wird einer Lösung von Acrylatklebstoff (z. B. Duro-Tak 326-1753, National Starch & Chemical) und ggf. weiteren Hilfsstoffen in Ethylacetat und n-Hexan zugefügt und homogen gemischt. Die Lösung wird mit einer Naßschichtdicke von 450 *µ*m mittels eines Ziehrakels auf eine silikonisierte Polyesterfolie (z. B. Hostaphan, Hoechst) aufgetragen und bei 50 °C 1 h getrocknet. Die getrocknete Schicht wird mit einer Polyesterfolie (Abziehfolie bzw. Release Liner) laminiert. Aus dem Laminat werden mittels einer geeigneten Stanze TTS mit Flächen von 10, 20, 30 und 40 cm² ausgestanzt. Die in der folgenden Tabelle aufgeführten Zusammensetzungen wurden nach obenstehendem Beispiel hergestellt und die in-vitro-Permeation durch isolierte Haut weiblicher Nacktmäuse gemessen.

### Erläuterungen zur Tabelle

- Tacrin-Base: Konzentration in der fertigen Matrix 20%;
- lipophiler Enhancer: Konzentration verschiedener lipophiler Enhancer, wie Copherol F 1300, Miglyol 840, Isopropylmyristat;
- hydrophiler Enhancer: Konzentration hydrophiler Lösemittel wie Propylenglycol, Glycerin, Transcutol;
- *µ*g/cm²/Zelle: in 48 h permeierte Substanzmenge

Tabelle 1 verdeutlicht auf eindrucksvolle Weise, wie die Kombination eines hydrophilen und lipophilen Enhancers eine überadditive Hautpermeation im Vergleich zu den Hautpermeationswerten des hydrophilen und lipophilen Enhancers allein bewirkt.

**Tabelle 1:**

| Enhancer | errechnete Hautpermeation der Kombination (*µ*g/cm²/48h) | tatsächlich gemessene Hautpermeation (*µ*g/cm²/48h) |
|---|---|---|
| Copherol 25 % | | 154,3 |
| Propylenglykol 25 % | | 320,0 |
| Copherol 25 % + Propylenglykol 25 % | 474,3 | 1196,0 |
| Migliol 840 25 % | | 221,6 |
| Migliol 840 25 % + Propylenglykol 25 % | 541,6 | 1761,7 |
| Glycerin 25 % | | 240,9 |
| Glycerin 25 % + Copherol 25 % | 395,2 | 519,1 |
| Isopropylmyristat 25 % | | 342,4 |
| Isoprylmyristat 25 % + Propylenglykol 25 % | 662,4 | 814,7 |
| Transcutol 25 % | | 116,1 |
| Coph. 25 % + Transcutol 25 % | 270,4 | 492,6 |

### Beispiel 2

Für die Klebemittelschicht wurde ein druckempfindliches Klebemittel auf Silikon-Basis verwendet (trimethyliertes Siliciumdioxid, das mit Polydimethylsiloxan mit endständigen Trimethylsiloxy-Gruppen behandelt worden ist; Schichtdicke trocken (SD) etwa 35 bis 45 *µ*m; Flächengewicht (FG) etwa 50 bis 60 g/m²). Eine Abdeckfolie aus PET (Dicke etwa 75 *µ*m; FG etwa 100 g/m²) wurde mit dem Silikon-Kleber mit Hilfe einer Beschichtungsanlage beschichtet. Auf die beschichtete Abdeckfolie wurde eine mikroporöse Polypropylenmembran (heißsiegelfähig; Dicke etwa 50 *µ*m; FG etwa 120 g/m²) derart aufkaschiert, so daß ein Laminat aus Abdeckfolie, Klebemittel und Membran hergestellt wurde. Danach wurde das Laminat mit Hilfe einer Siegelmaschine (mit Schweißring) mit einer Trägerfolie aus Polyester (aluminiumbedampft) mit Polyolefin-Siegelschicht (heißsiegelfähig; Dicke etwa 70 *µ*m) derart verschweißt, daß ein Spalt zum Einfüllen einer Wirkstofflösung zurückblieb. Das befüllbare transdermale therapeutische System (Leer-TTS) wurde beispielsweise mit einer Hamilton-Spritze oder mit einer Schlauchpumpe mit Kanüle mit der folgenden Wirkstofflösung gefüllt:

| Zusammensetzung der Wirkstofflösung pro TTS: | |
|---|---|
| | mg/TTS |
| Tacrin-Base | 40.00 |
| Propylenglykol | 180.00 |
| α-Tocopherol | 80.00 |
| Insgesamt | 300.00 |
| Füllmenge des Reservoirs: | 300.0 mg |

Nach dem Befüllen wurde der Befüllungsspalt verschweißt. Befüllte transdermale therapeutische Systeme wurden mit Hilfe einer Stanze ausgestanzt.

## Patentansprüche

1. Pflaster zur transdermalen Applikation mit einer äußeren Abdeckschicht, einer selbstklebenden Matrix und einer entfernbaren Schutzschicht, wobei die Matrix,
- Tacrin oder eines seiner pharmazeutisch verträglichen Salze als Wirkstoff neben
- einem hydrophilen schwerflüchtigen Lösungsmittel und/oder
- einem lipophilen schwerflüchtigen Lösungsmittel enthält.

2. Pflaster nach Anspruch 1, **gekennzeichnet** durch einen Gehalt von bis zu 70 Gew.-% (bezogen auf das Matrix-Gewicht) an einem Lösungsmittel aus der durch hydrophile schwerflüchtige Lösungsmittel, lipophile schwerflüchtige Lösungsmittel und deren Gemische gebildeten Gruppe.

3. Pflaster zur transdermalen Applikation mit einer äußeren Abdeckschicht, einem Reservoir, einem Klebeelement für den Hautkontakt des Pflasters und einer entfernbaren Schutzschicht, wobei das Reservoir
- Tacrin oder eines seiner pharmazeutisch verträglichen Salze als Wirkstoff neben
- einem Lösungsmittel aus der durch hydrophile schwerflüchtige Lösungsmittel, hydrophobe schwerflüchtige Lösungsmittel, einwertige C₂₋₄-Alkohole, Ethylenglykol und deren Gemische gebildeten Gruppe enthält.

4. Pflaster nach einem der vorhergehenden Ansprüche, **gekennzeichnet** durch eine Membran, insbesondere eine die Wirkstoffpermeation steuernde Membran.

5. Pflaster nach Anspruch 4, dadurch **gekennzeichnet**, daß das Reservoir durch die Abdeckschicht und die Membran oder durch eine Matrix gebildet wird.

6. Pflaster nach einem der vorhergehenden Ansprüche, **gekennzeichnet** durch eine Matrix auf Polyacrylatbasis, Silikon-basis oder Polyisobutylenbasis.

7. Pflaster nach Anspruch 3, 4, 5 und/oder 6, **gekennzeichnet** durch ein Klebeelement in Form einer das Reservoir (sofern keine Membran vorgesehen ist) oder einer die Membran vollständig oder an ihrer Peripherie ringförmig abdeckenden Schicht.

8. Pflaster nach Anspruch 3, 4, 5, 6 und/oder 7, **gekennzeichnet** durch ein Klebeelement aus einem druckempfindlichen Klebemittel auf Silikonbasis.

9. Pflaster nach einem der vorhergehenden Ansprüche, **gekennzeichnet** durch einen Gehalt an α-Tocopherol oder an einem α-Tocopherol-Derivat.

10. Pflaster nach einem der vorhergehenden Ansprüche, **gekennzeichnet** durch Propylenglykol, 1,2-Pentandiol, Glycerin, hydrophiles Cetiol oder Transcutol als hydrophiles schwerflüchtiges Lösungsmittel.

11. Pflaster nach einem der vorhergehenden Ansprüche, **gekennzeichnet** durch Copherol, Migliol, i-Propylmyristat oder lipophiles Cetiol als lipophiles schwerflüchtiges Lösungsmittel.

12. Pflaster nach einem der vorhergehenden Ansprüche, **gekennzeichnet** durch einen Gehalt an hydrophilem schwerflüchtigen Lösungsmittel und lipophilem schwerflüchtigen Lösungsmittel.

## Claims

1. Patch for transdermal application having an outer cover layer, a self-adhesive matrix and a removable protective layer, wherein the matrix comprises
- tacrine or a pharmaceutically acceptable salt thereof as active ingredient in addition to
- a hydrophilic poorly volatile solvent and/or
- a lipophilic poorly volatile solvent.

2. Patch according to claim 1, characterised by a content of up to 70 % by weight (based on the matrix weight) of a solvent from the group formed by hydrophilic poorly volatile solvents, lipophilic poorly volatile solvents and mixtures thereof.

3. Patch for transdermal application having an outer cover layer, a reservoir, an adhesive element for contact of the patch with the skin and a removable protective layer, wherein the reservoir comprises
- tacrine or a pharmaceutically acceptable salt thereof as active ingredient in addition to
- a solvent from the group formed by hydrophilic poorly volatile solvents, hydrophobic poorly volatile solvents, monohydric C₂-C₄ alcohols, ethylene glycol and mixtures thereof.

4. Patch according to any one of the preceding claims, characterised by a membrane, especially a membrane that controls the permeation of active ingredient.

5. Patch according to claim 4, characterised in that the reservoir is formed by the cover layer and the membrane or by a matrix.

6. Patch according to any one of the preceding claims, characterised by a polyacrylate-based, silicone-based or polyisobutylene-based matrix.

7. Patch according to claim(s) 3, 4, 5 and/or 6, characterised by an adhesive element in the form of a layer that covers the reservoir (where no membrane is provided) or the membrane completely or around its periphery in a ring-shape.

8. Patch according to claim(s) 3, 4, 5, 6 and/or 7, characterised by an adhesive element made of a pressure-sensitive silicone-based adhesive.

9. Patch according to any one of the preceding claims, characterised by a content of α-tocopherol or an α-tocopherol derivative.

10. Patch according to any one of the preceding claims, characterised by propylene glycol, 1,2-pentanediol, glycerol, hydrophilic cetiol or transcutol as hydrophilic poorly volatile solvent.

11. Patch according to any one of the preceding claims, characterised by copherol, miglyol, isopropyl myristate or lipophilic cetiol as lipophilic poorly volatile solvent.

12. Patch according to any one of the preceding claims, characterised by a content of hydrophilic poorly volatile solvent and lipophilic poorly volatile solvent.

## Revendications

1. Pansement pour application transdermique ayant une couche externe de recouvrement, une matrice auto-adhésive et une couche de protection amovible, dans lequel la matrice contient
- de la tacrine ou un de ses sels acceptables sur le plan pharmaceutique en tant que principe actif, en plus de
- un solvant hydrophile peu volatil et/ou
- un solvant lipophile peu volatil.

2. Pansement selon la revendication 1, caractérisé par une teneur allant jusqu'à 70 % en poids (par rapport au poids de la matrice) d'un solvant choisi dans le groupe formé par les solvants hydrophiles peu volatils, les solvants lipophiles peu volatils et leurs mélanges.

3. Pansement pour application transdermique ayant une couche de recouvrement externe, un réservoir, un élément adhésif pour le contact de la peau avec le panse-ment et une couche protectrice amovible, dans lequel le réservoir contient
- de la tacrine ou un de ses sels acceptables sur le plan pharmaceutique en tant que principe actif, en plus de
- un solvant choisi dans le groupe formé par les solvants hydrophiles peu volatils, les solvants hydrophobes peu volatils, les mono-alcools en C₂ à C₄, l'éthylèneglycol et leurs mélanges.

4. Pansement selon l'une quelconque des revendications précédentes, caractérisé par une membrane, en particulier une membrane régulant l'infiltration du principe actif.

5. Pansement selon la revendication 4, caractérisé en ce que le réservoir est formé par la couche de recouvre ment et la membrane ou par une matrice.

6. Pansement selon l'une quelconque des revendications précédentes, caractérisé par une matrice à base ce polyacrylate, à base de silicone ou à base de polyiso-butylène.

7. Pansement selon les revendications 3, 4, 5 et/ou 6, Caractérisé par un élément adhésif sous forme d'une couche recouvrant. le réservoir (dans la mesure où aucune membrane n'est prévue) ou d'une couche recouvrant complètement la membrane ou de forme annulaire sur sa périphérie.

8. Pansement selon les revendications 3, 4, 5, 6 et/ou 7, caractérisé par un élément adhésif constitué d'une colle auto-adhésive à base de silicone.

9. Pansement selon l'une quelconque des revendications précédentes, caractérisé par une teneur en α-tocophérol ou en un dérivé d'α-tocophérol.

10. Pansement selon l'une quelconque des revendica Lions précédentes, caractérisé par le propylèneglycol, le 1,2-pontanediol, la glycérine, le Cetiol ou Transcutol hydrophiles en tant que solvant hydrophile peu volatil.

11. Pansement selon l'une quelconque des revendications précédentes, caractérisé par le Copherol, le Migliol, le myristate d'isopropyle ou le Cetiol lipophile en tant que solvant lipophile peu volatil.

12. Pansement selon l'une quelconque des revendications précédentes, caractérisé par une teneur en solvant hydrophile peu volatil et en solvant lipophile peu volatil.
